# EUROPÄISCHE PATENTSCHRIFT

(11) **EP 0 632 994 B1**
(45) Veröffentlichungstag und Bekanntmachung des Hinweises auf die Patenterteilung: **07.01.1999**
(21) Anmeldenummer: 94108334.7
(22) Anmeldetag: 30.05.1994
(51) Int. Cl.: A61B 6/03, A61B 6/14, A61B 6/00

(54) **Röntgendiagnostikeinrichtung zur Erstellung von Röntgenaufnahmen von Körperteilen eines Patienten**
X-ray diagnostic device for producing X-rays of body parts of a patient
Appareil de radio diagnostic pour produire des radiographies des parties du corps d'un patient

(30) Priorität: 06.07.1993 DE 4322483
(43) Veröffentlichungstag der Anmeldung: 11.01.1995
(73) Patentinhaber: Sirona Dental Systems GmbH & Co.KG, 64625 Bensheim (DE)
(72) Erfinder: Heubeck, Erich, D-64625 Bensheim (DE); Döbert, Michael, D-64653 Lorsch (DE); Günther, Werner, D-64625 Bensheim (DE); Plötz, Josef, Dr. Dipl.-Phys., D-64625 Bensheim (DE); Schulze-Ganzlin, Ulrich, Dipl.-Ing., D-64653 Lorsch (DE)
(74) Vertreter: Isenbruck, Günter, Dr.

(56) Entgegenhaltungen:
- EP-A- 0 166 567
- DE-A- 3 930 022

## Beschreibung

Die Erfindung bezieht sich auf eine Röntgendiagnostikeinrichtung zur Erstellung von Panorama-Schichtaufnahmen vom Kiefer eines Patienten, von cephalometrischen Aufnahmen oder von Mammographieaufnahmen eines Patienten.

Aus EP-0 166 567 A3 ist ein Röntgengerät bekannt, mit dem ausschließlich Übersichtsaufnahmen vom Thorax eines Patienten, dagegen keine Schichtaufnahme von einem Körperteil gemacht werden können. Der von einer Strahlenquelle ausgehende fächerförmig ausgeblendete Röntgenstrahl durchdringt den Körper eines Patienten und trifft dann auf mehrere Zeilendetektoren, die der Strahlenquelle gegenüberliegend angeordnet sind. Die Strahlenquelle kann um ihren Fokus um eine Vertikalachse geschwenkt werden. Synchron dazu wird die Detektorzeile ebenfalls um eine horizontale Achse geschwenkt. Die Zeilendetektoren bestehen aus mehreren CCD-Elementen, die die Röntgenstrahlung in elektrische Signale umwandeln. Die Detektorzeilen können zwar bei dieser bekannten Einrichtung aus CCD-Elementen gebildet werden, die im TDI-Modus betrieben werden; die Anordnung der CCD-Elemente zu einem Array und deren Betriebsweise ist jedoch so, daß stets ein Summensignal von allen durchstrahlten Körperteilen gebildet wird. Damit wird ein Durchstrahlungsprofil erzeugt, in welchem alle im Durchstrahlungsbereich befindlichen Körperteile berücksichtigt sind, was letztlich bedeutet, daß man in der Auswertung der Signale eine Übersichtsaufnahme vom Thorax, dagegen keine Schichtaufnahmen bekommt.

Zur Erstellung beispielsweise von Schichtaufnahmen vom Kiefer eines Patienten, von cephalometrischen oder Mammographie-Aufnahmen ist es nach dem Stand der Technik notwendig und üblich, nach dem zu durchstrahlenden Körperteil eine Filmkassette mit dem zu belichtenden Röntgenfilm anzuordnen. Für cephalometrische (Ceph-)Aufnahmen ist es aus EP-A-0 262 522 bekannt, einen Schädelhalter vorzusehen, der mittels eines Trägers an einem Auslegerarm befestigt ist. Dieser ist wiederum im notwendigen Abstand an einem Stativ, an dem auch der Röntgenstrahler angeordnet ist, befestigt. Am Schädelhalter befindet sich eine Halterung zur Aufnahme der Röntgenfilmkassette in der für Ceph-Aufnahmen notwendigen Größe.

Für Panorama-Schichtaufnahmen (PAN-Aufnahmen) ist es erforderlich, eine andere Filmkassette mit anderem Filmformat vorzusehen, die synchron, aber gegenläufig, zur Bewegung der Strahlenquelle verstellt wird. Bei einem bekannten solchen Gerät (EP-0 229 308 A1) ist eine an einem Laufwagen eines Stativs angeordnete Dreheinheit vorgesehen, an dem die Strahlenquelle und die diametral dazu eine Halterung für eine den Röntgenfilm aufnehmende Filmkassette gehaltert sind. Um mit dieser Röntgendiagnostikeinrichtung auch Ceph-Aufnahmen machen zu können, ist am Laufwagen des Stativs der eingangs erwähnte Auslegerarm zu adaptieren, an dem Kopfhalte- und Positioniereinrichtung sowie die Halterung für die für Ceph-Aufnahmen geeignete Filmkassette befestigt sind.

Der im Anspruch 1 angegebenen Erfindung liegt die Aufgabe zugrunde, eine Verbesserung dahingehend zu schaffen, die eingangs genannten unterschiedlichen Aufnahmen ohne Film und Filmkassette, stattdessen durch Gewinnung von Sensordaten mit wirtschaftlich vertretbarem Aufwand machen zu können.

Die erfindungsgemäß vorgeschlagene Verwendung einer Zeilendetektorkamera mit gleich ausgebildeter Halterung ermöglicht es, auf besonders wirtschaftliche Weise sowohl PAN- als auch Ceph- als auch Mamma-Aufnahmen in Slot-Technik zu erstellen. Die Zeilendetektorkamera kann waagerecht oder hochkant angeordnet sein. Sie enthält vorteilhafterweise einen Steckanschluß, der je nach Bedarf an einen geeigneten Steckplatz eines PAN-, Ceph- oder Mamma-Gerätes angeschlossen werden kann. Die applizierte Röntgendosis ist durch die Dicke des Strahlfächers und dessen Verfahrgeschwindigkeit bestimmt. Bei Ceph-Aufnahmen wird beispielsweise der Kopf des stehenden oder sitzenden Patienten von oben nach unten mit einem seitlich geöffneten Strahlfächer überstrichen, der stets genau auf den waagerecht angeordneten Sensor trifft und dessen Länge und Breite abdeckt. Bei vertikaler Anordnung kann die Strahlenquelle motorisch in waagerechter Richtung geschwenkt oder translatorisch bewegt werden; alternativ kann bei feststehender Strahlenquelle, die Sekundärblende synchron zum Zeilendetektor geschwenkt oder translatorisch bewegt werden.

Besondere Vorteile sind darin zu sehen, daß für PAN- oder Ceph-Aufnahmen ein und dieselbe Zeilendetektorkamera verwendet werden kann, welche bei der einen Aufnahmeart vorzugsweise senkrecht, bei der anderen vorzugsweise waagerecht gehaltert wird. Damit läßt sich der Herstellungsaufwand für das Gesamtgerät erheblich reduzieren.

Weitere Vorteile ergeben sich aus den Unteransprüchen und der nachfolgenden Beschreibung eines Ausführungsbeispieles.

Es zeigen:
Figur 1 eine Prinzipdarstellung eines zahnärztlichen Röntgendiagnostikgerätes zur Erstellung von PAN-Aufnahmen,
Figur 2 das Gerät nach Figur 1, modifiziert zur Erstellung von Ceph-Aufnahmen,
Figur 3 die für Ceph-Aufnahmen vorgesehene Vorrichtung in schaubildlicher Darstellung,
Figur 4 die Vorrichtung nach Figur 3 in Frontansicht, teilweise im Schnitt,
Figur 5 eine Ausführungsform einer Zeilendetektorkamera mit zugehörigem Halter in schaubildlicher Explosionsdarstellung,
Figur 6 die Zeilendetektorkamera im Querschnitt, entlang der Linie VI-VI in Figur 5,
Figur 7 ein Blockschaltbild zur Ansteuerung der Verstellmotoren,
Figuren 8 und 9 eine Prinzipdarstellung weiterer Ausführungsformen eines Röntgendiagnostikgerätes zur Erstellung von Ceph-Aufnahmen,
Figur 10 eine schematisierte Darstellung des Strahlenganges.
Figur 11 eine weitere vorteilhafte Ausführungsform.

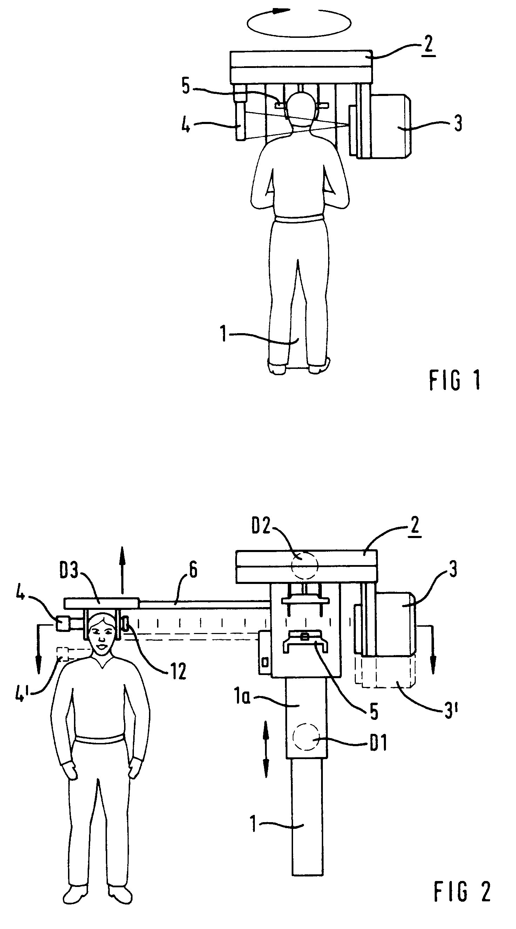

Die Figur 1 zeigt in einer Prinzipdarstellung ein zehnärztliches Röntgendiagnostikgerät zur Erstellung von Panorama-Schichtaufnahmen, nachfolgend kurz mit PAN-Aufnahmen bezeichnet. Das Gerät enthält eine in der Höhe verstellbare Tragsäule 1, an der eine Dreheinheit 2 gehaltert ist, die Träger einerseits einer Röntgenstrahlenquelle 3 und andererseits diametral dazu einer Röntgenzeilenkamera 4 ist. Mit 5 ist eine (erste) Kopfhalte- und Positioniereinrichtung bezeichnet, mit der in bekannter Weise der Patientenkopf in einer definierten Position fixiert werden kann. Aufbau sowie Verstellmöglichkeiten der Dreheinheit und der Kopfhalte und Positioniereinrichtung sind bekannt und beispielsweise in der EP-0 229 308 beschrieben.

Die Figur 2 zeigt das gleiche Grundgerät, bestehend aus höhenverstellbarer Tragsäule 1, Dreheinheit 2 und Röntgenstrahler 3, ergänzt jedoch durch eine am Gerät adaptierbare Vorrichtung, mit der sich Schädelfernaufnahmen, nachfolgend kurz Ceph-Aufnahmen, erstellen lassen. Bevor die genannte Vorrichtung näher erläutert wird, sei noch erwähnt, daß die Tragsäule 1 mittels eines Antriebes D1 in der angegebenen Pfeilrichtung höhenverstellbar ausgebildet ist, daß weiterhin in bekannter Weise die Dreheinheit 2 mittels eines oder mehrerer Antriebe D2 gedreht und geschwenkt werden kann, um eine PAN-Aufnahme machen zu können. Einzelheiten hierzu ergeben sich aus der bereits genannten EP-0 229 308.

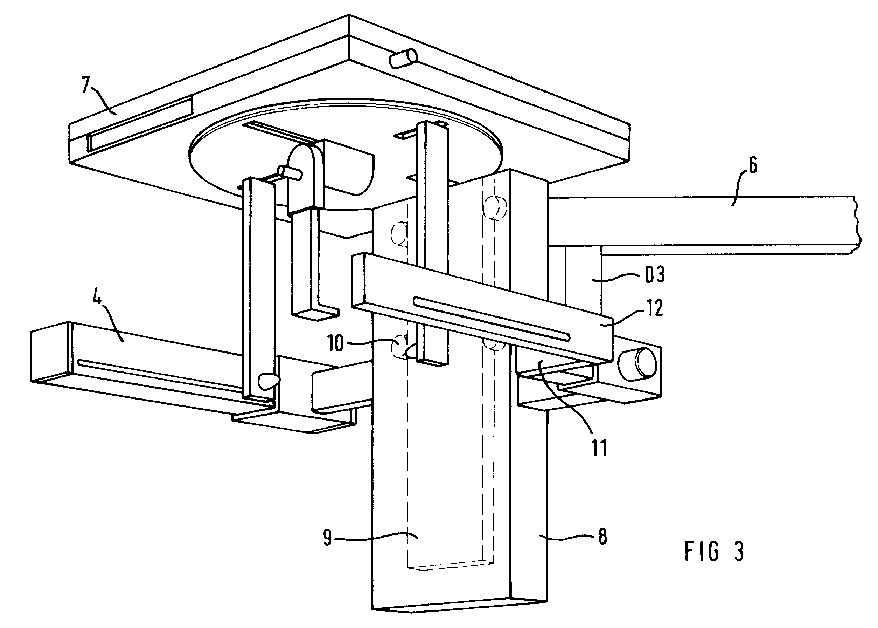

Die Figur 3 zeigt die vorgenannte Vorrichtung zur Erstellung von Ceph-Aufnahmen in einer schaubildlichen Darstellung.

Am höhenverstellbaren Teil 1a der Tragsäule 1 (Fig. 2) ist ein Auslegerarm 6 befestigt, der eine (zweite) Kopfhalte- und Positoniereinrichtung 7 trägt. Der Auslegerarm 6 umfaßt ein Gehäuse 8, an dem ein Schwert 9, welches die Kopf-halte und Positioniereinrichtung trägt, mit Hilfe von im Gehäuse 8 angeordneten Führungsrollen 10 verstellbar gelagert ist. Die Zeilenkamera 4 ist mittels eines Querträgers 11 mit einer Vorblende 12 verbunden, die dazu dient, den an sich schon in bekannter Weise von der der Röntgenstrahlenquelle 3 benachbarten Sekundärblende begrenzten Fächerstrahl nochmals exakt auf die Schlitzbreite und -länge der nachfolgend noch näher erläuterten Zeilenkamera zu justieren.

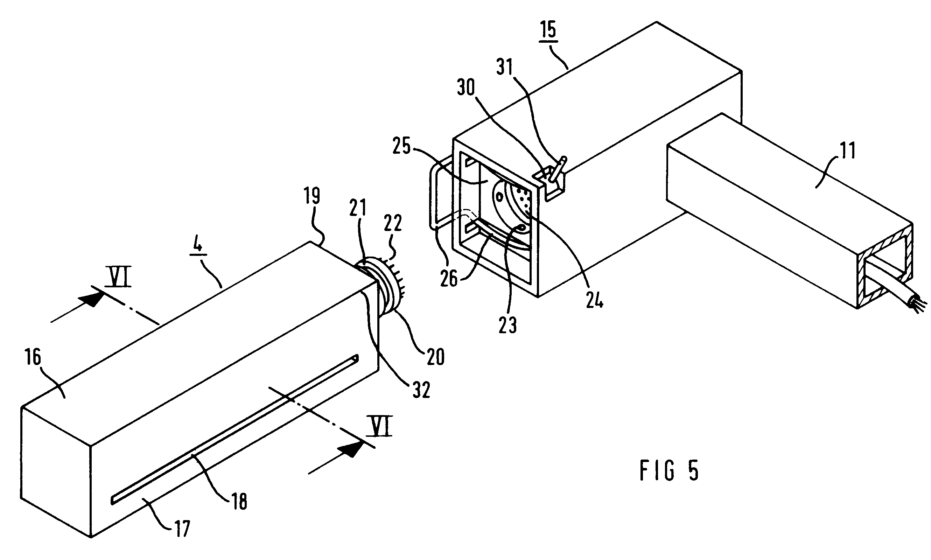

Im Gegensatz zum Ausführungsbeispiel nach Figur 1 ist die Zeilenkamera bei der Ausführung nach Figur 2 nicht vertikal, sondern waagerecht angeordnet. Eine entsprechend ausgebildete Halterung ist in Figur 5 dargestellt.

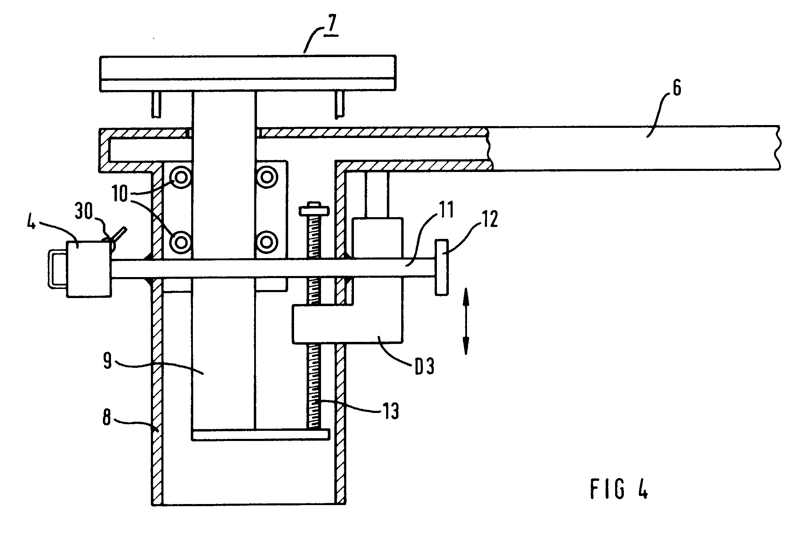

Wie aus Figur 4, die die Vorrichtung in Frontansicht und teilweise im Schnitt zeigt, ersichtlich, befindet sich am Schwert 9, welches die Kopfhalte- und Positioniereinrichtung trägt, eine Gewindespindel 13, die mit einem allgemein mit D3 bezeichneten Getriebemotor zusammenwirkt. Der Getriebemotor D3 ist entweder am Gehäuse 8 oder am Tragarm 6 befestigt. Wie später noch näher erläutert, wird mit Hilfe der aufgezeigten Verstellanordnung mit dem Antrieb D3 erreicht, daß, wenn die Röntgenstrahlenquelle 3 zusammen mit der Zeilenkamera 4 in der Vertikalen bewegt wird, die Kopfhalte- und Positioniereinrichtung 7 während der Ceph-Aufnahme effektiv keine Bewegung ausführt, d.h. ortsfest im Raum gehalten wird.

Die Figur 5 zeigt in einer schaubildlichen Explosionsdarstellung einerseits die Zeilenkamera 4 und andererseits eine mit 15 bezeichnete Halterung, die im vorliegenden Anwendungsfalle für die Ausführung nach Figur 2 am Querträger 11 befestigt ist. Im Falle der Version nach Figur 1 (für PAN-Aufnahmen) ist eine gleich ausgebildete Halterung (ohne Querträger 11) senkrecht an der Dreheinheit 2 (Figur 1) befestigt.

Die Zeilenkamera 4 enthält ein längliches Gehäuse 16, welches im Ausführungsbeispiel aus einem Vierkantrohr besteht und in der vorderen, der Strahlenquelle 3 zugewandten Seitenfläche 17 einen Schlitz 18 aufweist. Der Schlitz 18 befindet sich im unteren Drittel der Seitenfläche 17, wodurch die Zeilenkamera in eine vergleichsweise tiefe Ausgangsposition (sh. gestrichelte Darstellung in Fig. 2) gefahren werden kann.

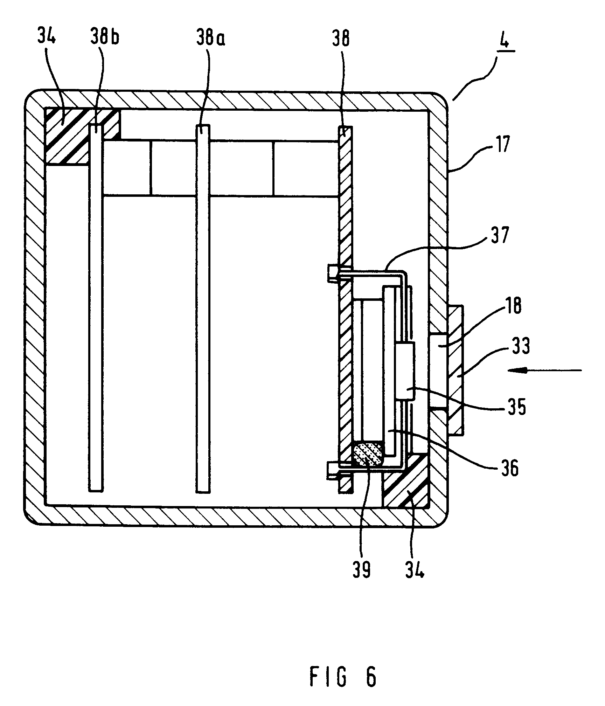

Wie aus Figur 6 noch näher hervorgeht, befindet sich hinter dem Schlitz 18 im Innern des Profilrohres 16 ein strahlenempfindlicher Zeilendetektor, z.B. in Form eines CCD-Sensors. An der einen Stirnseite 19 befindet sich ein zapfenförmiges Anschlußelement 20, welches mechanische und elektrische Anschlußmittel für eine elektrische und mechanische Verbindung mit dem Halter 15 aufweist. Die mechanischen Anschlußmittel enthalten eine Ringnut 21, die mit einer Kugelrastung 23 zusammenwirkt. Die elektrischen Anschlußmittel bestehen aus einem Mehrstiftstecker 22, der mit einer Steckbuchse 24 im Halter 15 zusammenwirkt. Die Steckstifte 22 sind mit dem bereits erwähnten Zeilendetektor und einer weiteren, im Inneren der Zeilenkamera 4 befindlichen Elektronik verbunden. Der Halter 15 ist so aufgebaut, daß bei aufgesetztem Zeilendetektor die Stirnseite 19 der Zeilenkamera 4 einer stirnseitigen Anschlußfläche 25 der Halterung 15 gegenübersteht.

Damit das Lösen der Zeilenkamera 4 von der Halterung 15 erleichtert ist, insbesondere ein Verkanten und damit die Gefahr einer Beschädigung der hochempfindlichen elektrischen Kontakte vermieden wird, ist eine Auswurfeinrichtung 26 vorgesehen. Diese besteht in der vorliegenden Ausführungsform aus einem Bügel, der in einem Schlitz der Gehäusewandung des Halters 15 nach außen geführt ist. Wird der Bügel bei aufgesetzter Zeilenkamera betätigt, drücken anliegende Bügelteile gegen die Stirnfläche 19 und üben so eine zentrische Kraft auf die Fläche aus, wodurch die Verbindung leicht gelöst werden kann.

Mit 30 ist eine Zentriereinrichtung bezeichnet, die einen exzentrisch im Gehäuse der Halterung gelagerten Hebel 31 enthält. Nach Einsetzen der Zeilenkamera 4 in den Halter 15 wird der Exzenterhebel 31 betätigt, wodurch eine Fläche das Exzenters auf die in der Figur mit 32 bezeichnete Kante des Gehäuses drückt und dieses in definierter, reproduzierbarer Position hält. Obgleich im vorliegenden Ausführungsbeispiel das Gehäuses einteilig ausgebildet ist, kann das Gehäuse auch mehrteilig ausgeführt sein, wobei der eine, den Detektor tragende Gehäuseteil dann in der vorgenannten Weise zentriert wird. Damit kann der Detektor unabhängig vom Kameragehäuse und dessen etwaigen Montage- und Fertigungstoleranzen in bezug auf den Halter fixiert werden.

Aus Figur 6, die einen Schnitt entlang der Linie VI-VI in Figur 5 zeigt, geht der prinzipielle Aufbau der Zeilenkamera hervor. Das Gehäuse ist lichtdicht ausgebildet; der Schlitz 18 ist stirnseitig von einer lichtdichten, aber röntgenstrahlendurchlässigen Kunststoffplatte 33 bedeckt. Dahinter befindet sich im Innern ein mit einer vorgeschalteten Szintillationsschicht und gegebenenfalls mit einer zwischengeschalteten Faseroptik versehener CCD-Sensor 35. Der CCD-Sensor 35 kann ein- oder mehrteilig und vorteilhafterweise als Sensormatrix aus amorphem Silizium ausgebildet sein. Ein metallischer Halter 37 verbindet den Träger 36 und das CCD-Element 35 mit einer Platine 38. Flexible Kontaktstreifen 39, z.B. aus mit Goldfasern versehenem Silicon, bewirken den elektrischen Kontakt zwischen Sensor 35 und Platine 38. Die Platine 38 enthält sämtliche, zur Ansteuerung des CCD-Sensors unmittelbar erforderlichen Bauelemente. Gegebenenfalls sind im Gehäuse noch weitere Platinen 38a, 38b angeordnet. Die von der (den) Platine(n) 38 (38a, 38b) abgehenden Leitungen führen zu den bereits erwähnten Steckstiften 22 (Fig. 5). Mit 34 sind stoßabsorbierende Elemente bezeichnet, die den Detektor 35 und die Steuerplatinen 38, 38a, 38b im Gehäuse 'schwimmend' lagern. Damit lassen sich die hochempfindlichen und teueren Teile bei einem unbeabsichtigten Herabfallen der Kamera vor Bruch bzw. Lösen der Kontaktverbindungen weitgehend schützen.

Wie eingangs bereits erwähnt, ist für PAN-Aufnahmen (Fig. 1) und Ceph-Aufnahmen (Fig. 2) das gleiche Grundgerät und ein und dieselbe Kamera verwendbar. Um die für eine Ceph-Aufnahme nötige Bildgröße zu erreichen, hat die Zeilenkamera vorteilhafterweise einen entsprechend längeren Sensor. Die Zeilenkamera kann so je nach Bedarf entweder am Ceph- oder am PAN-Halter angebracht werden. Zur Halterung der Zeilenkamera am Halter 15 sind verschiedene Möglichkeiten denkbar. Anstelle der gezeigten Kugelrastung kann auch eine Bajonettverbindung vorgesehen sein. Desgleichen kann anstelle eines Vierkantprofiles eine andere äußere Formgestaltung für das Gehäuse der Zeilenkamera vorgesehen sein.

Zum Aufnahmeprinzip wird folgendes angemerkt:

Eine PAN-Schichtaufnahme wird in der Weise erzielt, daß die beim Überstreichen des aufzunehmenden Objekts (Kiefer) gewonnenen Signale in dem zweidimensional auflösenden Detektor aufaddiert werden, wobei das Aufaddieren der Signale - falls ein CCD-Sensor verwendet wird - bereits auf dem Sensor durchgeführt werden kann, indem dieser im TDI-Modus betrieben wird. Durch diese besondere Betriebsart wird die Funktion eines bewegten Filmes nachgebildet, indem die durch Belichtung erzeugten Ladungspakete im CCD-Element entsprechend weitergetaktet werden, während ständig neue Ladungen hinzukommen. Die Taktimpulse für den TDI-Betrieb werden aus den sonst für den Filmkassettenantrieb erforderlichen Schrittmotorimpulsen abgeleitet. Alternativ ist auch ein Aufaddieren in einer späteren Signalverarbeitungsstufe möglich.

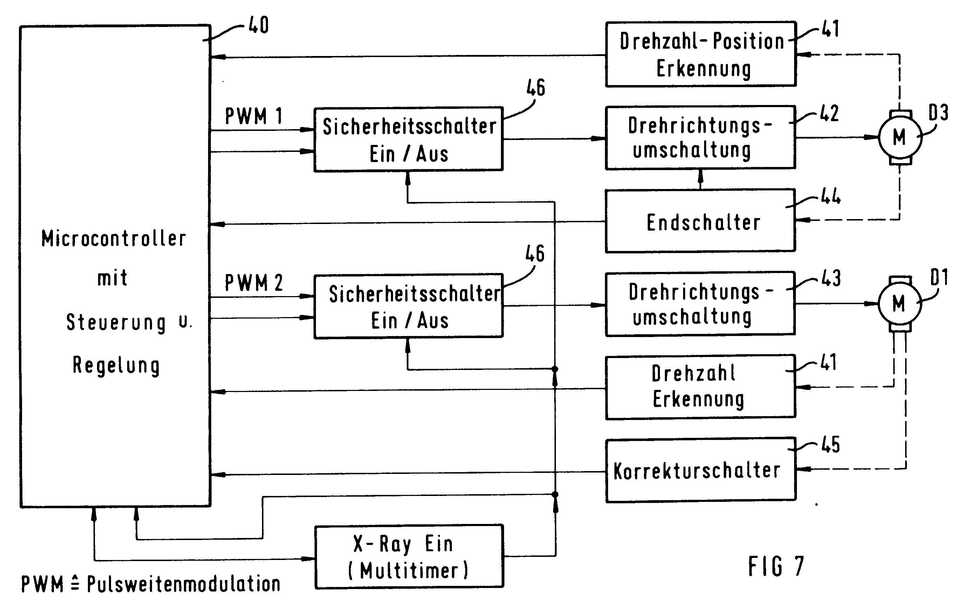

Die Ceph-Aufnahme läuft ebenfalls in Slot-Technik ab. Der Kopf eines stehenden (oder sitzenden) Patienten wird je nach Anordnung der Zeilenkamera von oben nach unten (bei waagerechter Anordnung) oder von links nach rechts (bei senkrechter Anordnung) vice versa mit einem Strahlfächer überstrichen. Dieser Strahlfächer trifft, justiert durch die bereits genannte Vorblende 11, genau auf den waagerecht angeordneten Schlitz des CCD-Sensors. Mit Hilfe des Antriebs D1 verfährt man nun das gesamte Gerät, also Röntgenstrahlenquelle 3 mit Primär- und Sekundärblende sowie Zeilenkamera 4 mit Sensor, gemeinsam von einer Ausgangsposition aus in der Vertikalen (sh. Pfeile in Fig. 2). Gleichzeitig wird die Kopfhalte- und Positioniereinrichtung 7 mit Hilfe des Antriebs D3 in Gegenrichtung gefahren, wobei die beiden Bewegungen so aufeinander abgestimmt sind, daß der Patientenkopf räumlich fixiert, d.h. ortsfest, bleibt. Die Steuerung der beiden Antriebsmotoren D1 und D3 erfolgt entsprechend dem Blockschaltbild nach Figur 7 über einen Mikroprozessor 40. Den beiden Antrieben sind Drehzahlerkennungssensoren 41, Drehrichtungsumschalter 42 sowie End- bzw. Korrekturschalter 44, 45 zugeordnet. Die über Pulsweitenmodulation erfolgte Steuerung enthält weiterhin Sicherheitsschalter 46. Eine Auswerteelektronik des Mikro-Controllers 40 erkennt, an welcher Halterung (PAN- oder Ceph-Gerät) die Kamera befestigt ist. Wird eine Ceph-Aufnahme angewählt, fährt der Antriebsmotor D3 in die Ausgangsposition, beispielsweise in die untere Verstellposition (gestrichelte Position in Fig. 2). In dieser Position spricht der Endschalter 44 an. Mittels der Höhenverstellung der Tragsäule 1 kann nun die Kopfhaltepositoniereinrichtung auf die Patientengröße eingestellt werden. Während der Ceph-Aufnahme verfährt der Antriebsmotor D3 die Kopfpositioniereinrichtung 7 nach oben, während gleichzeitig der Antriebsmotor D1 für die Tragsäule nach unten fährt. Die beiden Antriebe werden dabei so geregelt, daß die Differenz der Verstellgeschwindigkeiten gleich Null ist. Dadurch ist sichergestellt, daß der Abstand der Ohroliven und damit der Kopfpositionierung zum Fußboden konstant bleibt. Die Aufnahme ist beendet, wenn der Endschalter 44 oder ein Systemtakt-Zähler (TDI-Takt-Zähler) die obere Endlage erkennt.

Der TDI-Takt für den CCD-Sensor wird z.B. vom Antriebsmotor D1, der für die Höhenverstellung der Tragsäule vorgesehen ist, abgeleitet. Alternativ kann er auch aus den Signalen eines Positionszählers, der die Verstellung der Tragsäule direkt mißt, gewonnen werden. Der TDI-Modus dient hier nicht, wie bei einer PAN-Aufnahme, um eine Verwischung und damit eine Schichtaufnahme zu erzeugen, sondern dazu, die volle Breite des Sensors zur Bildentstehung auszunutzen. Hier entspricht also der TDI-Betrieb einem Film, der relativ zum Schlitz bewegt wird und relativ zum Patienten feststeht.

Die nachfolgenden Figuren zeigen vorteilhafte Varianten zu der in Figur 2 dargestellten Ausführungsform anhand einer Ansicht von oben (Draufsicht). Im Gegensatz zu der Ausführung nach Figur 2, bei der die Zeilenkamera 4 horizontal angeordnet ist, ist bei den Varianten nach Figuen 8 und 9 die Zeilenkamera 4 vertikal angeordnet; demnach wird auch von der Primärblende und der Vorblende ein vertikales gefächertes Strahlenbündel auf den CCD-Sensor gegeben.

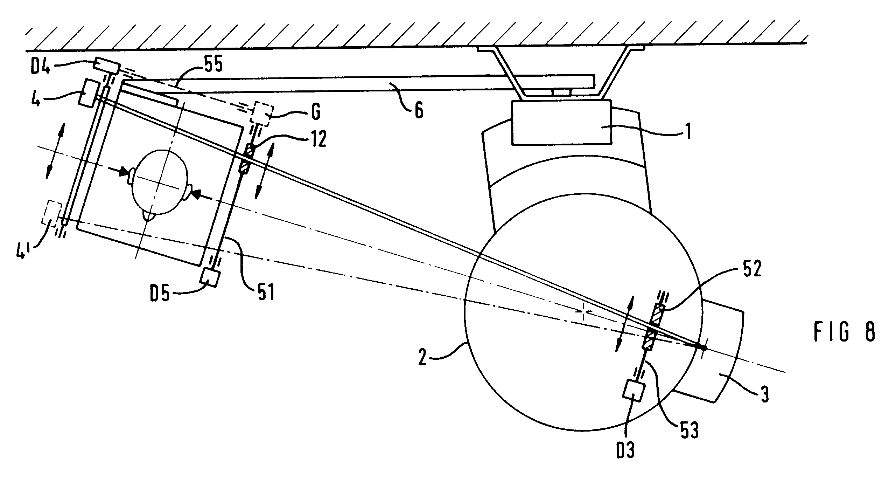

Bei der Ausführung nach Figur 8 ist sowohl die Zeilenkamera 4 als auch die Vorblende 12 in Längsführungen 50, 51 geführt, die motorisch z.B. mittels eines gemeinsamen Antriebes D4 oder, unter Zwischenschaltung eines Untersetzungsgetriebes (G) durch getrennte Antriebe D4 und D5 bewegt werden. Die Primärblende 52 ist ebenfalls mittels einer Längsführung 53 in Pfeilrichtung bewegbar. Zur Verstellung ist hier ein Antrieb D6 vorgesehen. Die Längsführungen 50, 51 und 53 können in bekannter Weise als motorisch angetriebene Gewindespindelantriebe ausgebildet sein. Die Winkelgeschwindigkeiten für die Antriebe D4, D5 und D6 sind dann, wenn jeweils getrennte Antriebe vorgesehen sind, gleich. Wenn für Längsführungen 50 und 51 ein gemeinsamer Antrieb vorgesehen werden soll, müssen die beiden Längsführungen über eine weitere Spindel 55 unter Zwischenschaltung eines geeigneten Untersetzungsgetriebes (G) miteinander gekoppelt sein.

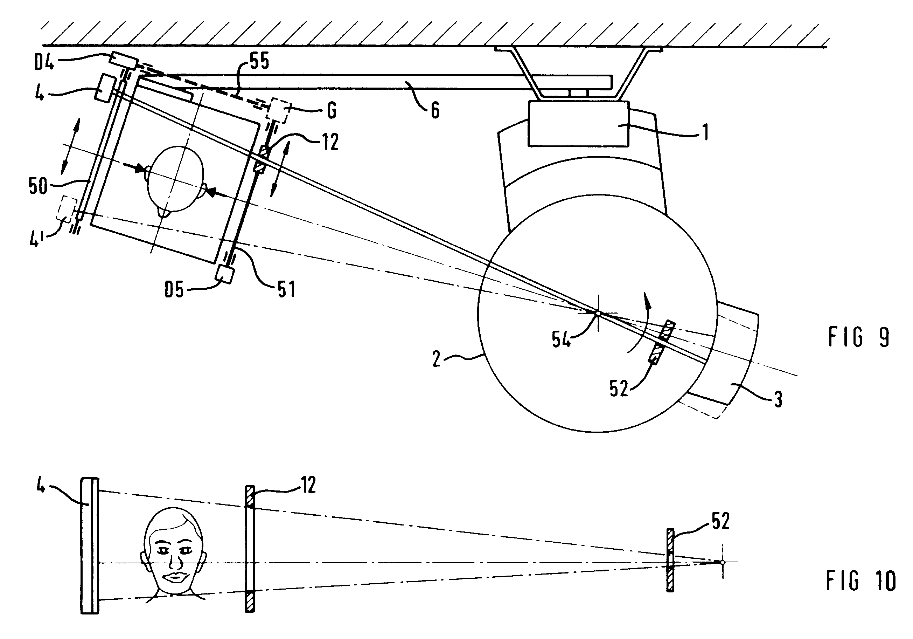

Bei der Ausführung gemäß Figur 9 wird die Primärblende nicht bewegt. Sie läuft vielmehr starr mit der Dreheinheit 2 um deren Drehmittelpunkt 54 um. Auch bei dieser Version ist es denkbar, die beiden Längsführungen 50 und 51 durch eine weitere Spindel und ein geeignetes, dazwischen gesetztes Untersetzungsgetriebe G miteinander zu synchronisieren.

Die Figur 10 zeigt eine mögliche Ausführung einer Strahlausrichtung, nämlich eine asymmetrische Einstellung des Strahles in bezug auf das zu durchstrahlende Objekt. Anstelle der asymmetrischen Einstellung ist auch eine symmetrische Ausrichtung des Fächerstrahls möglich.

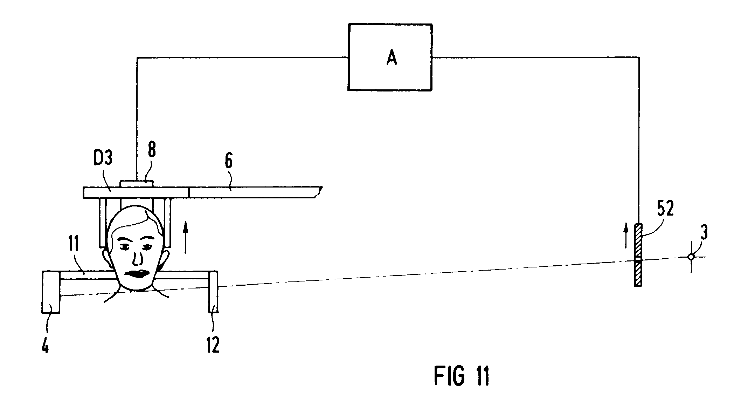

Die Figur 11 zeigt eine weitere vorteilhafte Ausführungsform, bei der die Kamera 4, wie in Figur 2 gezeigt, horizontal angeordnet und über einen Querträger 11 mit der Vorblende 12 verbunden ist. Kamera 4 und Vorblende 12 werden gemeinsam über einen Antrieb A mit der Primärblende 52 motorisch verstellt, wobei die Verstellung, wie beschrieben, im TDI-Modus erfolgt. Die Strahlenquelle 3 bleibt bei dieser Ausführungsform während der Verstellbewegung von Kamera und Primärblende ortsfest.

## Patentansprüche

1. Röntgendiagnostikeinrichtung zur Erstellung von Panorama-Shichtaufnahmen vom Kiefer eines Patienten, von cephalometrischen Aufnahmen oder von Mammographieaufnahmen eines Patienten, unter Verwendung einer diametral gegenüber einer Strahlenquelle (3) angeordneten Zeilendetektor-Kamera (4), die zur Erstellung der Panorama-Schichtaufnahmen und der Mammographieaufnahmen patientennah und zur Erstellung der cephalometrischen Aufnahmen patientenfern montiert ist und die einen hinter einer schlitzförmigen Öffnung (18) angeordneten Röntgenstrahlen-Detektor (35) beinhaltet, dessen Breite der Breite bzw. Länge des aufzunehmenden Körperteils angepaßt ist, wobei Verstellmittel (D1, D4) vorhanden sind, welche die Zeilendetektor-Kamera gegenüber dem Körperteil so verstellen, daß die Schlitzöffnung entlang des Körperteils bewegt wird, wobei der von der Strahlenblende (52, 12) der Strahlenquelle (3) begrenzte Fächerstrahl synchron zur Kamerabewegung mitbewegt wird und bei der für die vorgenannten unterschiedlichen Aufnahmen gleich ausgebildeten Halterungen (15) für die Zeilendetektor-Kamera (4) vorgesehen sind.

2. Röntgendiagnostikeinrichtung nach Anspruch 1, bei der die Zeilendetektor-Kamera (4) so gehaltert ist, daß die Schlitzöffnung (18) waagerecht verläuft und die Verstellbewegung in senkrechter Richtung erfolgt (Fig. 2).

3. Röntgerdiagostikeinrichtung nach Anspruch 2, bei der die Zeilerdetektor-Kamera (4) einem Wandadapter zugeordnet ist, der Verstellmittel für eine Höhenverstellung eines Halters für die Kamera beinhaltet.

4. Röntgendiagnostikeinrichtung nach Anspruch 2, bei der die Zeilendetektor-Kamera (4) einem Auslegerarm (6) zugeordnet ist, der höhenverstellbar an einem die Strahlenquelle (3) tragenden Stativ (1) gehaltert ist (Fig. 2).

5. Röntgendiagnostikeinrichtung nach Anspruch 1, bei der für cephalometrische Aufnahmen die Zeilendetektor-Kamera (4) so gehaltert ist, daß die Schlitzöffnung (18) vertikal verläuft und die Verstellbewegung in waagerechter Richtung erfolgt.

6. Röntgendiagnostikeinrichtung nach Anspruchs 5, bei der die Strahlenquelle (3) fest und die ihr vorgeschaltete Primärblende (52) synchron zur Verstellung der Kamera verstellt wird.

7. Röntgendiagnostikeinrichtung nach Anspruch 5, bei der die Strahlenquelle (3) verstellbar angeordnet ist und diese zusammen mit der Primärblende (52) um den Drehmittelpunkt (54) einer die Strahlenquelle tragenden Dreheinheit (2) verstellbar angeordnet ist.

8. Röntgendiagnostikeinrichtung nach einem der Ansprüche 1 bis 7, welche zur Erstellung von einerseits Panorama-Schichtaufnahmen (PAN-Aufnahmen) vom Kiefer eines Patienten und andererseits von Fernaufnahmen (Ceph-Aufnahmen) vom Schädel eines Patienten folgende Elemente beinhaltet:
- ein Stativ (1), mit einem höhenverstellbaren Tragteil (1a);
- eine Dreheinheit (2) mit einer Röngenstrahlenquelle (3) und einer ersten Kopfhalte- und Positioniereinrichtung (5),
- einen ersten Halter (15) für eine Zeilendetektor-Kamera (4) zur Erstellung der PAN-Aufnahmen,
- einen Auslegerarm (6), an dem eine zweite Kopfhalte- und Positioniereinrichtung (7) angeordnet ist, sowie
- einen zweiten Halter (15) mit baugleichem Anschlußteil (24) für die alternative Halterung der vorgenannten Zeilendetektor-Kamera (4) zur Erstellung der Ceph-Aufnahmen,
wobei der erste Halter so angeordnet ist, daß der Zeilendetektor mit seiner Längsausdehnung vertikal ausgerichtet ist und gemeinsam mit der Strahlenquelle (3) um den Patientenkopf herum bewegbar ist, während der zweite Halter so angeordnet ist, daß die Zeilendetektor-Kamera mit ihrer Längsausdehnung horizontal ausgerichtet ist und in bezug auf die Strahlenquelle in vertikaler Ebene verstellbar angeordnet ist.

9. Röntgendiagnostikeinrichtung nach Anspruch 8, bei der die zweite Kopfhalte- und Positioniereinrichtung (7) an einem ortsfesten, geräteexternen Teil (Wand) und die Zeilendetektor-Kamera (4) am höhenverstellbaren Auslegerarm (6) befestigt sind.

10. Röntgendiagnostikeinrichtung nach Anspruch 8, bei der die zweite Kopfhalte- und Positioniereinrichtung (7) am höhenverstellbaren Auslegerarm (6) angeordnet ist, und zwischen dem Auslegerarm und der zweiten Kopfhalte- und Positioniereinrichtung eine Einrichtung (10, 13, D3) vorgesehen ist, welche die Bewegung des höhenverstellbaren Tragteils (1a) während der Ceph-Aufnahme kompensiert.

11. Röntgendiagnostikeinrichtung nach einem der Ansprüche 1 bis 10, bei der die Zeilendetektor-Kamera (4) ein Anschlußteil (20) aufweist, welches Anschlußmittel für eine lösbare mechanische und elektrische Verbindung mit dem Halter (15) beinhaltet.

12. Röntgendiagnostikgerät nach Anspruch 1, bei der die Zeilendetektor-Kamera (4) so gehaltert ist, daß die Schlitzöffnung (18) horizontal verläuft und die Verstellbewegung von Primärblende (52); Vorblende (12) und Zeilendetektor-Kamera (4) in vertikaler Richtung erfolgt, indem diese Teile gemeinsam mittels eines Antriebs (A) verstellt werden, wobei während der Verstellbewegung die Strahlenquelle (3) ortsfest bleibt.

13. Röntgendiagnostikeinrichtung nach einem der Ansprüche 1-12, bei der der Röntgenstrahlen-Detektor (35) ein CCD-Sensor ist, der aus ein oder mehreren Zeilen aufgebaut ist und eine vorgesetzte Szintillationsschicht enthält.

14. Röntgendiagnostikeinrichtung nach Anspruch 13, bei der der CCD-Sensor (35) eine zwischengeschalteten Faseroptik enthält.

15. Röntgendiagnostikeinrichtung nach einem der Ansprüche 1-12, bei der der Röntgenstrahlen-Detektor (35) eine Sensormatrix aus a-Si und eine Szintillationsschicht enthält.

## Claims

1. Radiodiagnostics device for producing panoramic tomograms of the jaw of a patient, cephalometric radiograms or mammograms of a patient, using a line-detector camera (4), which is arranged diametrically opposite a radiation source (3) and is mounted close to the patient for producing panoramic tomograms and mammograms and is mounted at a distance from the patient for producing the cephalometric radiograms and contains an X-ray detector (35), which is arranged behind a slit-like opening (18) and the width of which is matched to the width or length of the part of the body that is to be radiographed, with there being adjusting means (D1, D4) which adjust the line-detector camera with respect to the part of the body in such a way that the slit opening is moved along the part of the body, the fan-shaped ray, which is delimited by the radiation diaphragm (52, 12) of the radiation source (3), being moved synchronously with the camera movement, and in which there are provided holding devices (15) for the line-detector camera (4) that are constructed identically for the different radiograms mentioned above.

2. Radiodiagnostics device according to claim 1, in which the line-detector camera (4) is held in such a way that the slit opening (18) extends horizontally and the adjusting movement takes place in the vertical direction (Figure 2).

3. Radiodiagnostics device according to claim 2, in which there is allocated to the line-detector camera (4) a wall adapter, which contains adjusting means for a vertical adjustment of a holder for the camera.

4. Radiodiagnostics device according to claim 2, in which there is allocated to the line-detector camera (4) a cantilever arm (6) which is held in a vertically adjustable manner on a stand (1) which supports the radiation source (3) (Figure 2).

5. Radiodiagnostics device according to claim 1, in which, for cephalometric radiograms, the line-detector camera (4) is held in such a way that the slit opening (18) extends vertically and the adjusting movement takes place in the horizontal direction.

6. Radiodiagnostics device according to claim 5, in which the radiation source (3) is fixed and the primary diaphragm (52) connected in front of it is adjusted synchronously with the adjustment of the camera.

7. Radiodiagnostics device according to claim 5, in which the radiation source (3) is arranged so as to be adjustable, and is arranged in a manner such that, together with the primary diaphragm (52), it is adjustable about the centre of rotation (54) of a rotation unit (2) which supports the radiation source.

8. Radiodiagnostics device according to one of the claims 1 to 7, which, in order to produce panoramic tomograms (PAN radiograms) of the jaw of a patient on the one hand and tele-radiograms (ceph-radiograms) of the skull of a patient on the other hand, contains the following elements:
- a stand (1) having a vertically adjustable supporting portion (1a);
- a rotation unit (2) having an X-ray source (3) and a first head-supporting and positioning device (5);
- a first holder (15) for a line-detector camera (4) for producing the PAN radiograms;
- a cantilever arm (6), on which there is arranged a second head-supporting and positioning device (7); and also
- a second holder (15) having an identically constructed connecting piece (24) for the alternative holding device of the above-mentioned line-detector camera (4) for producing the ceph-radiograms,
with the first holder being arranged in such a way that the line detector is oriented in a manner such that its longitudinal extent is vertical and can be moved together with the radiation source (3) about the head of the patient, while the second holder is arranged in such a way that the line-detector camera is oriented in a manner such that its longitudinal extent is horizontal and is arranged so as to be adjustable in the vertical plane with respect to the radiation source.

9. Radiodiagnostics device according to claim 8, in which the second head-supporting and positioning device (7) is fastened on a stationary portion (wall) outside the device and the line-detector camera (4) is fastened on the vertically adjustable cantilever arm (6).

10. Radiodiagnostics device according to claim 8, in which the second head-supporting and positioning device (7) is arranged on the vertically adjustable cantilever arm (6), and provided between the cantilever arm and the second head-supporting and positioning device is a device (10, 13, D3) which compensates for the movement of the vertically adjustable carrying portion (1a) during the ceph-radiogram.

11. Radiodiagnostics device according to one of the claims 1 to 10, in which the line-detector camera (4) has a connecting piece (20) which contains connection means for a detachable mechanical and electrical connection to the holder (15).

12. Radiodiagnostics device according to claim 1, in which the line-detector camera (4) is held in such a way that the slit opening (18) extends horizontally and the adjusting movement of primary diaphragm (52), outer diaphragm (12) and line-detector camera (4) takes place in the vertical direction by adjusting these parts jointly by means of a drive (A), with the radiation source (3) remaining stationary during the adjusting movement.

13. Radiodiagnostics device according to one of the claims 1 to 12, in which the X-ray detector (35) is a CCD sensor, which is made up of one or more lines and contains a scintillation layer which is placed in front.

14. Radiodiagnostics device according to claim 13, in which the CCD sensor (35) contains an intermediate fibre optics.

15. Radiodiagnostics device according to one of the claims 1 to 12, in which the X-ray detector (35) contains a sensor matrix consisting of a-Si and a scintillation layer.

## Revendications

1. Installation de radiodiagnostic destinée à effectuer des clichés stratigraphiques panoramiques de la mâchoire d'un patient, des clichés céphalométriques ou des clichés mammographiques d'un patient en utilisant une caméra à détecteur horizontal (4), diamétralement opposée à une source de rayonnement (3), qui est montée à proximité du patient pour effectuer les clichés stratigraphiques panoramiques et les clichés mammographiques et à distance du patient pour effectuer les clichés céphalométriques, et qui comporte un détecteur de rayons X (35), disposé derrière un orifice (18) en forme de fente, dont la largeur est adaptée à la largeur ou, selon le cas, à la longueur de la partie du corps à radiographier, dans laquelle il existe des organes de déplacement (D1, D4) qui déplacent la caméra à détecteur horizontal par rapport à la partie du corps de telle manière que la fente se déplace le long de la partie du corps, dans laquelle le rayon en forme d'éventail délimité par le diaphragme d'irradiation (52, 12) de la source de rayonnement (3) se déplace de manière synchronisée en même temps que le mouvement de la caméra et dans laquelle il est prévu des supports (15) identiques destinés à la caméra à détecteur horizontal (4) pour effectuer les différents clichés mentionnés ci-dessus.

2. Installation de radiodiagnostic selon la revendication 1, dans laquelle la caméra à détecteur horizontal (4) est maintenue de telle manière que la fente (18) s'étend horizontalement et que le mouvement de déplacement a lieu dans la direction verticale (figure 2).

3. Installation de radiodiagnostic selon la revendication 2, dans laquelle la caméra à détecteur horizontal (4) est associée à un adaptateur mural qui comporte des organes de déplacement servant au déplacement en hauteur d'un support destiné à la caméra.

4. Installation de radiodiagnostic selon la revendication 2, dans laquelle la caméra à détecteur horizontal (4) est associée à une potence (6) qui est maintenue, de manière mobile en hauteur, par un statif (1) supportant la source de rayonnement (3) (figure 2).

5. Installation de radiodiagnostic selon la revendication 1, dans laquelle la caméra à détecteur horizontal (4) est maintenue, pour des clichés céphalométriques, de telle manière que la fente (18) s'étend verticalement et que le mouvement de déplacement a lieu dans la direction horizontale.

6. Installation de radiodiagnostic selon la revendication 5, dans laquelle la source de rayonnement (3) est fixe et dans laquelle le diaphragme primaire (52) placé devant cette dernière se déplace de manière synchronisée avec le mouvement de la caméra.

7. Installation de radiodiagnostic selon la revendication 5, dans laquelle la source de rayonnement (3) est disposée de manière mobile et dans laquelle cette dernière, de même que le diaphragme primaire (52), est disposée de manière mobile autour du point central de pivotement (54) d'un dispositif pivotant (2) supportant la source de rayonnement.

8. Installation de radiodiagnostic selon l'une des revendications 1 à 7 qui comporte, afin d'effectuer des clichés stratigraphiques panoramiques (clichés PAN) de la mâchoire d'un patient d'une part et des clichés à distance (clichés CEPH) d'autre part :
- un statif (1) ayant un élément porteur (1a),
- un dispositif pivotant (2) ayant une source de rayonnement (3) et un premier dispositif de support de la tête et de positionnement (5),
- un premier support (15) de caméra à détecteur horizontal (4) pour effectuer des clichés PAN,
- une potence (6) sur laquelle est disposé un second dispositif de support de la tête et de positionnement (7), ainsi que
- un second support (15), ayant une pièce de raccordement (24) identique, offrant l'autre possibilité de fixation de la caméra à détecteur horizontal (4) mentionnée ci-dessus pour effectuer les clichés CEPH,
le premier support étant disposé de telle sorte que le détecteur horizontal soit dirigé verticalement dans son étendue longitudinale et qu'il puisse se déplacer, en même temps que la source de rayonnement (3), autour de la tête du patient, le second support étant quant à lui disposé de telle sorte que la caméra à détecteur horizontal soit dirigée horizontalement dans son étendue longitudinale et soit disposée de manière mobile dans le plan vertical par rapport à la source de rayonnement.

9. Installation de radiodiagnostic selon la revendication 8, dans laquelle le second dispositif de support de la tête et de positionnement (7) est fixé à un élément immobile et extérieur à l'appareil (un mur) et dans laquelle la caméra à détecteur horizontal (4) est fixée à la potence (6) mobile en hauteur.

10. Installation de radiodiagnostic selon la revendication 8, dans laquelle le second dispositif de support de la tête et de positionnement (7) est disposé sur la potence (6) mobile en hauteur et dans laquelle il est prévu, entre la potence et le second dispositif de support de la tête et de positionnement, un dispositif (10, 13, D3) qui compense le déplacement de l'élément porteur (1a) mobile en hauteur pendant le cliché CEPH.

11. Installation de radiodiagnostic selon l'une des revendications 1 à 10, dans laquelle la caméra à détecteur horizontal (4) possède une pièce de raccordement (20) qui comporte des organes de raccordement pour l'établissement d'une liaison mécanique et électrique réversible avec le support (15).

12. Appareil de radiodiagnostic selon la revendication 1, dans lequel la caméra à détecteur horizontal (4) est maintenue de telle manière que la fente (18) s'étend horizontalement et que le mouvement de déplacement du diaphragme primaire (52), du diaphragme avant (12) et de la caméra à détecteur horizontal (4) a lieu dans la direction verticale du fait que ces éléments sont déplacés ensemble au moyen d'une commande (A), la source de rayonnement (3) restant fixe pendant le mouvement de déplacement.

13. Installation de radiodiagnostic selon l'une des revendications 1 à 12, dans laquelle le détecteur de rayons X (35) est un capteur à couplage de charge (capteur CCD) qui est constitué d'une ou de plusieurs lignes et comporte une couche de scintillation placée devant.

14. Installation de radiodiagnostic selon la revendication 13, dans laquelle le capteur à couplage de charge (35) comporte une optique sur fibres intercalée.

15. Installation de radiodiagnostic selon l'une des revendications 1 à 12, dans laquelle le détecteur de rayons X (35) comporte une matrice en silicium amorphe et une couche de scintillation.
